# EUROPEAN PATENT APPLICATION

(11) **EP 1 938 802 A1**
(43) Date of publication of application: **02.07.2008**
(21) Application number: 06301289.2
(22) Date of filing: 22.12.2006
(51) Int. Cl.: A61K 9/127, A61K 47/48, A61K 48/00, A61P 19/02

(54) **Interfering RNAs targeting pro-inflammatory cytokines**

(71) Applicant: INSERM (Institut National de la Santé et de la Recherche Médicale), 75654 Paris Cedex 13 (FR)
(72) Inventor: Apparailly, Florence, 34000 Montpellier (FR); Khoury, Maroun, 34090 Montpellier (FR)
(74) Representative: Chajmowicz, Marion

(57) **Abstract**

The invention relates to a pharmaceutical composition comprising interfering RNAs (iRNAs) silencing IL-1β, IL-6, IL-18 and/or IL-17, in combination with a lipopolyamine, and to the use of the composition for preventing or treating an inflammatory disorder such as rheumatoid arthritis.

SEQUENCE LISTING

<110>  INSERM

<120>  interfering RNAs targeting pro-inflammatory cytokines

<130>  B0535

<160>  8

<170>  PatentIn version 3.3

<210>  1
<211>  21
<212>  DNA
<213>  Artificial

<220>
<223>  IL-1b siRNA

<400>  1
ucaacaagau agaagucaat t                                                21


<210>  2
<211>  21
<212>  DNA
<213>  Artificial

<220>
<223>  IL-1b siRNA

<400>  2
uaauugacuu caccauggat t                                                21


<210>  3
<211>  21
<212>  DNA
<213>  Artificial

<220>
<223>  IL6 siRNA

<400>  3
cuaccaaacu ggauauaaut t                                                21


<210>  4
<211>  21
<212>  DNA
<213>  Artificial

<220>
<223>  IL6 siRNA

<400>  4
augcucuccu aacagauaat t                                                21


<210>  5
<211>  19
<212>  DNA
<213>  Artificial

<220>
<223>  IL6 siRNA

<400>  5
agacatgtaa caagagtaa                                                    19

<210>  6
<211>  21
<212>  DNA
<213>  Artificial

<220>
<223>  IL18 siRNA

<400>  6
caacacgcuu uacuuuauat t                                                 21

<210>  7
<211>  21
<212>  DNA
<213>  Artificial

<220>
<223>  IL18 siRNA

<400>  7
uucgaggaua ugacugauat t                                                 21

<210>  8
<211>  19
<212>  DNA
<213>  Artificial

<220>
<223>  IL17 siRNA

<400>  8
aaaggtcctc agattacta                                                    19

## Description

The present invention relates to a pharmaceutical composition comprising interfering RNAs (iRNAs) silencing IL-1β, IL-6, IL-18 and/or IL-17 useful for treating an inflammatory disorder such as rheumatoid arthritis.

### Background of the invention:

Rheumatoid arthritis (RA) is the most frequent inflammatory rheumatism affecting almost 1% of the world's population. This autoimmune disease is characterized by chronic inflammation within the joint tissue infiltrated by activated immune cells and by synovial hyperplasia, leading to cartilage and bone destruction after several years. The evolution results in significant morbidity, disability, and increased mortality. Although the causes of RA are not fully understood, TNF-α plays an important role in RA pathogenesis as evidenced by experimental and clinical data. Indeed, transgenic mice over-expressing TNF-α develop spontaneous, severe, erosive arthritis1, several therapies blocking the TNF-α have successfully been used in experimental arthritis -Bloquel, et al, 2004, Gould et al, 2004; Kim et al, 2004) and TNF-neutralizing agents administered to patients with long-lasting active RA were found to be beneficial (Taylor et al, 2005). Nevertheless, 30% of patients do not respond to anti-TNF biotherapies, efficiency can be lost along the treatment and disease relapses when stopping the therapy. The mechanism involved in the lack of response to anti-TNF is still unknown but evidence of an increased risk of serious infections and malignancies associated with the long-term anti-TNF antibody therapies6 support the development of alternative molecularly targeted therapies that might enhance specificity and clinical efficiency, as well as overcome the problem of resistance.

RNA interference (RNAi) is a naturally occurring gene silencing mechanism used by mammalian cells to control the expression of endogenous gene in a sequence-specific manner at the mRNA level, and is of great interest for developing therapies that are based on inhibition of function (Caplen 2004, Mello, 2004). Small interfering RNAs (siRNA) of about 21 nucleotides in length can be introduced into the cell cytoplasm for specific silencing of the target RNA in a sequence-dependent manner. Adopted as a standard experimental tool, RNAi has proven to be highly successful in reducing target gene expression, but important tasks still remain for in vivo applications, such as optimisation of the intracellular delivery and siRNA stability, as well as investigation of their therapeutic potential for clinically relevant gene targets. As innovative therapeutic agents, siRNAs entered for the first time in a Phase I trial in patients with age-related macular degeneration in 2004 (Karagiannis and El-Osta, 2005). The intra-vitreal injection of a siRNA silencing the VEGFR1 has shown to be safe, well tolerated, with no evidence of clinical toxicity, as well as angiogenesis regression and stabilization of the visual acuity in all patients.

The feasibility of using siRNA in vivo as therapeutic tool in RA was first reported following electro-transfer of siRNAs targeting TNF-α in knee joints of arthritic mice (Shiffelers et al, 2005) or rat (Inoue et al, 2005). More recently, it was shown that weekly systemic injection of a cationic liposome formulated with anti-TNF-α siRNA and carrier DNA in collagen-induced arthritic mice leads to a local and systemic TNF-α inhibition, associated with a complete protection from arthritis (Khoury et al, 2006).

Although TNF-α plays a key role in RA, other pro-inflammatory cytokines have been implicated and are targeted by current biotherapies (Dinarello et al, 2002). Firstly, IL-1b and IL-18 belong to the IL-1 superfamily and their role in RA is well established. They are important cytokines which biological properties increase the expression of many pro-inflammatory genes (Ghayur et al, 1997). IL-18 functions primarily as a costimulant for Th1 cytokine production and thus induces TNF-α production by macrophages and synoviocytes, and its secretion is increased by IL-1b et TNF-α (Kohno et al, 1997; Tsutsui et al, 1996; Gracie et al, 1999). Moreover, IL-1b was shown to possess many of the biological properties of TNF-α on systemic and local inflammation, and on tissue remodelling in arthritis (Dayer et al, 1977; Dinarello et al, 1986). Secondly, IL-6 has been implicated in both local destructive and proliferative processes that accompany RA, as well as many of the systemic symptoms experienced by patients with active disease (Feldmann et al, 1996). A direct role of IL-6 in the pathogenesis of RA has been confirmed both in experimental models and clinic. Serum concentrations of IL-6 are elevated in patients with RA and frequently correlate with severity of clinical disease. It has been reported that the cytokine interleukin 17 (IL-17) stimulates epithelial, endothelial, and fibroblastic cells to secrete cytokines such as IL-6, IL-8, and granulocyte-colony-stimulating factor, as well as prostaglandin E2.

Finally, blocking IL-1β, IL-6, IL-17or IL-18 activities with monoclonal antibody or binding protein have shown therapeutic benefit in patients refractory to anti-TNF biotherapies (Choy et al, 2002, Illei et al, 2000, Mc Innes et al, 2003, Taylor et al, 2003) or led to significant CIA (collagen-induced arthritis) amelioration (Plater-Zyberk et al, 2001; Rohn et al, 2006).

### Summary of the invention:

The present invention now provides a new interfering RNA formulation for treating inflammatory disorders.

A subject of the invention is a pharmaceutical composition comprising an interfering RNA (iRNA) silencing at least one cytokine selected from the group consisting of IL-1β, IL-6, IL-18 and IL-17, in combination with a lipopolyamine, preferably RPR209120. The RNA is preferably small interfering RNA (siRNA).

Preferably at least two cytokines are silenced. More preferably the composition comprises a combination of iRNAs silencing IL-1β, IL-6, and IL-18.

In a preferred embodiment, the composition further comprises a neutral lipid, preferably dioleoylphosphatidylethanolamine (DOPE).

Preferably the composition further comprises DNA, as a carrier.

Another subject of the invention is the use of such composition, for the preparation of a medicament for preventing or treating an inflammatory disorder, in particular rheumatoid arthritis.

### Detailed description of the invention:

The inventors propose to target IL-1β, IL-6, IL-18 and/or IL-17, by using an innovative iRNA formulation, particularly useful for systemic inhibition in RA. The inventors showed that experimental arthritis can be prevented by intravenous delivery of separate pro-inflammatory cytokines siRNA complexed with a lipopolyamine, and optionally a neutral lipid. In the context of the present invention, these complexes are designated "lipoplexes".

According to the invention, these complexes, especially when applied intravenously, offer protection from RA and other inflammatory disorders, and provide therapeutic benefit when administered after disease onset.

The inventors have more particularly shown that separate administration of siRNA-lipoplexes silencing IL-1β, IL-6 or IL-18 delayed disease onset, decreased arthritis incidence and severity, and inhibited local and systemic pro-inflammatory gene expression. Importantly, the combined IL-1β, IL-6, IL-18 formulated siRNAs also provide a highly effective therapy for collagen-induced arthritis, with striking effects on joint damage assessed both at the radiological and histological levels, which are not reached by any of the single anti-cytokine siRNA formulations.

More interestingly for the clinical relevance of such a therapeutic approach, treatment of established arthritis with the mixed siRNA-lipoplexes decreased the frequency of arthritis, ameliorated symptoms and joint damage. The therapeutic effect on arthritis is associated with a striking reduction of two deleterious components of the disease, i.e. inflammatory and autoimmune responses. The siRNA-lipoplex tri-therapy strongly reduces inflammatory responses during CIA development by down-regulating the production of pro-inflammatory agents in inflamed joints, including IL-1β, IL-6, TNF-α, as well as chemokines such as MCP-1, which have been shown to exacerbate CIA. In addition, the combined siRNA-lipoplexes increase secretion of the IL-10 anti-inflammatory cytokine, which have been reported to ameliorate arthritic symptoms.

IL-17 is another cytokine which can be advantageously silenced.

### siRNAs:

IL-1β, IL-6, IL-18 and IL-17 are prominent pro-inflammatory cytokines in rheumatoid arthritis, secreted mainly by macrophages. The sequences of each of these cytokines are well-known. For instance the sequences can be found on Genebank or EMBL: hIL-1b (NM000576), hIL-6 (NM000600), hIL-17 (NM002190), hIL-18 (NM001562).

RNA interference (RNAi) is a mechanism involving double-stranded RNA (dsRNA) molecules and resulting in post-transcriptional sequence-specific silencing of gene expression.

It is a multistep process, involving in a first step the cleavage, through the action of the Dicer enzyme (a RNase III endonuclease), of large dsRNAs into 21-23 ribonucleotides-long double stranded effector molecules called small interfering RNAs (siRNAs). These siRNAs duplexes bind to a protein complex to form the RNA-induced silencing complex (RISC). The RISC specifically recognises and cleaves the endogenous mRNAs containing a sequence complementary to one of the siRNA strands.

The term "interfering RNAs" means any RNA which is capable of down-regulating the expression of the targeted cytokine. It encompasses smal interfering RNA (siRNA), double-stranded RNA (dsRNA), micro-RNA (miRNA), and short hairpin RNA (shRNA) molecules.

The iRNAs can be chemically synthesized or expressed from a viral or non-viral vector or enzymatically synthesized.

The term "siRNA" as used herein refers to a double-stranded small interfering RNA unless otherwise noted. The siRNAs used to silence the transcription of a cytokine selected from the group consisting of IL-1β, IL-6, IL-18 and IL-17 may comprise a double stranded RNA wherein one strand of the RNA is complementary to the RNA of the cytokine. In another embodiment, a siRNA molecule comprises a double stranded RNA wherein one strand of the RNA comprises a portion of a sequence of the cytokine RNA. In yet another embodiment, a siRNA molecule comprises a double stranded RNA wherein both strands of RNA are connected by a non-nucleotide linker.

In one embodiment, a single strand component of a siRNA molecule is from about 14 to about 50 nucleotides in length. In another embodiment, a single strand component of a siRNA molecule is about 14, 15, 16, 17, 18, 19, 20, 21, 22, 23, 24, 25, 26, 27, or 28 nucleotides in length.

For example, the siRNA for silencing mouse and human IL-1β may be a double strand nucleic acid having the sequences as shown in SEQ ID NO :1 or SEQ ID NO :2. The sequence is given for the sense strand.

The siRNA for silencing mouse IL-6 may be a double strand nucleic acid having the sequence as shown in SEQ ID NO :3 or SEQ ID NO :4. Of note these sequences have been designed to silence the mouse cytokine. One skilled in the art can design other sequences targeting the human cytokine. Another siRNA for silencing human IL-6 may be SEQ ID NO:5.

The siRNA for silencing mouse and human IL-18 may be a double strand nucleic acid having the sequence as shown in SEQ ID NO :6 or SEQ ID NO :7.

An siRNA for silencing mouse IL-17 may be a double strand nucleic acid having the sequence as shown in SEQ ID NO:8, for silencing human IL-17.

In a particular embodiment, the iRNA includes a 3'-overhang that ranges from 1 to about 6 nucleotides. As used herein, a "3'overhang" refers to at least one unpaired nucleotide extending from the 3' end of an iRNA sequence. The 3' overhang can include ribonucleotides or deoxyribonucleotides or modified ribonucleotides or modified deoxyribonucleotides. The 3' overhang is preferably from 1 to about 5 nucleotides in length, more preferably from 1 to about 4 nucleotides in length and most preferably from about 2 to about 4 nucleotides in length. The 3' overhang can occur on the sense or antisense sequence, or on both sequences of an iRNA agent.

The iRNAs can be unmodified or chemically-modified nucleic acid molecules.. The use of a chemically-modified iRNA can improve one or more properties of an iRNA through increased resistance to degradation, increased specificity to target moieties, improved cellular uptake, and the like. For example, the iRNA can include a non-nucleotide moiety, such as a 2'-O-methyl (2'-OMe) pyrimidine nucleotide, 2'-deoxy nucleotide (e.g., deoxy-cytodine), 2'-deoxy-2'-fluoro (2'-F) pyrimidine nucleotide, 2'-O-methoxyethyl (2'-O-MOE), 2'-O-aminopropyl (2'-O-AP), 2'-O--N-methylacetamido (2'-O-NMA), 2'-O-dimethylaminoethlyoxyethyl (2'-DMAEOE), 2'-O-dimethylaminoethyl (2'-O-DMAOE), 2'-O-dimethylaminopropyl (2'-O-AP), 2'-hydroxy nucleotide, or a 2'-ara-fluoro nucleotide, or a locked nucleic acid (LNA), extended nucleic acid (ENA), hexose nucleic acid (HNA), cyclohexene nucleic acid (CeNA), ribo-difluorotoluyl, 5-allyamino-pyrimidines, or 5-Me-2'-modified pyrimidines. A 2' modification is preferably a 2'-OMe modification, and more preferably, a 2'-fluoro modification.

The iRNA can also include a nucleobase modification, such as a cationic modification, such as a 3'-abasic cationic modification. The cationic modification can be, e.g., an alkylamino-dT (e.g., a C6 amino-dT), an allylamino conjugate, a pyrrolidine conjugate, a pthalamido a hydroxyprolinol conjugate or an aminooxy conjugate, on one or more of the terminal nucleotides of the iRNA. An alkylamino-dT conjugate is preferably attached to the 3' end of the sense or antisense strand of an iRNA.

In a particular embodiment, the pharmaceutical composition of the invention may further comprise an iRNA for silencing TNF-α, e.g. as described in Khoury et al, 2006.

### The lipopolyamine:

The compositions of the invention comprise a lipopolyamine. The term lipopolyamine denotes an amphiphilic molecule comprising at least one hydrophilic polyamine region combined, via a so-called spacer region, with a lipophilic region. The polyamine region of lipopolyamines, which are cationically charged, is capable of combining reversibly with nucleic acid, which is negatively charged. This interaction compacts the nucleic acid greatly. The lipophilic region renders this ionic interaction insensitive to the external medium, by coating the nucleolipid particle formed with a lipid film. In compounds of this type, the cationic group may be represented by the L-5-carboxyspermine radical which contains four ammonium groups, two primary and two secondary.
lipopolyamines,

In a preferred embodiment, the lipopolyamine is a lipopolyamine in D, L or D,L form or its salts, represented by the general formula I 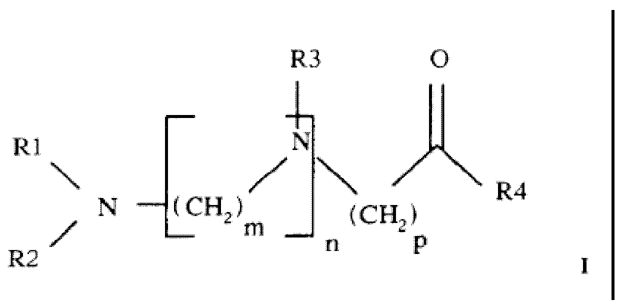 in which:
R1, R2 and R3 represent, independently of each other, a hydrogen atom or a group -(CH2)q-NRR' with
q able to range between 1, 2, 3, 4, 5 and 6, and doing so independently between the various groups R1, R2 and R3 and
R and R' representing, independently of each other, a hydrogen atom or a group --(CH2)q'-NH2, q being able to range between 1, 2, 3, 4, 5 and 6, and doing so is independently between the various groups R and R',
m, n and p represent, independently of each other, an integer which may vary between 0 and 6 with, when n is greater than 1, m able to take different values and R3 able to take different meanings within the general formula I, and
R4 represents a group of general formula II 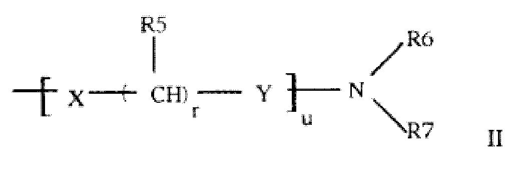 in which:
R6 and R7 represent, independently of each other, a hydrogen atom or a saturated or unsaturated C10 to C22 aliphatic radical with at least one of the two groups being other than hydrogen,
u is an integer chosen between 0 and 10 with, when u is an integer greater than 1, R5, X, Y and r able to have different meanings within the different units [X-(CHR5)r-Y]
X represents an oxygen or sulphur atom or an amine group which may or may not be monoalkylated,
Y represents a carbonyl group or a methylene group R5 represents a hydrogen atom or a side chain of a natural amino acid, which is substituted if necessary, and
r represents an integer ranging between 1 and 10 with, when r is equal to 1, R5 representing a side chain of a natural amino acid and, when r is greater than 1, R5 representing a hydrogen atom.

The expression "side chain" of a natural amino acid is understood in particular to denote chains containing amidinium units such as, for example, the side chain of arginine. As mentioned above, this chain may be substituted with saturated or unsaturated, linear, branched or cyclic C 1 to C24 aliphatic groups such as, for example, cholesteryl, arachidonyl or retinoyl radicals and mono- or polyaromatic groups such as, for example, benzyloxycarbonyl derivatives, benzyl ester derivatives and substituted or unsubstituted rhodaminyl derivatives.

These lipopolyamines of general formula (I) may be in the form of non-toxic and pharmaceutically acceptable salts. These non-toxic salts comprise salts with inorganic acids (hydrochloric acid, sulphuric acid, hydrobromic acid, phosphoric acid and nitric acid) or with organic acids (acetic acid, propionic acid, succinic acid, maleic acid, hydroxymaleic acid, benzoic acid, fumaric acid, methanesulphonic acid and oxalic acid) or with inorganic bases (sodium hydroxide, potassium hydroxide, lithium hydroxide and lime) or organic bases (tertiary amines such as triethylamine, piperidine and benzylamine).

In a most preferred embodiment, the lipopolyamine is RPR209120 which is

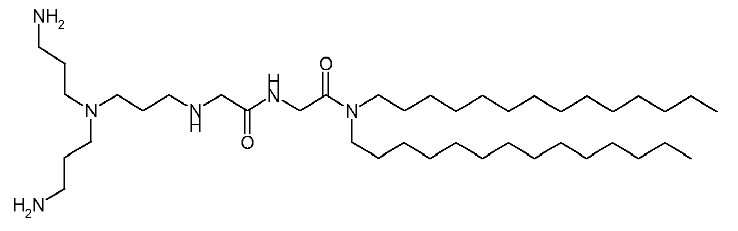

The synthesis of the lipopolyamine of formula I is shown in U.S.patent No.6,171,612, which further shows additional representatives of these lipopolyamines.

Any other targeting molecule, such as a lipid, may be added to the composition. For instance, one can target mannose receptors, e.g. by adding a neutral lipid which carry mannose residues, in the composition.

### Adjuvant:

In a particular embodiment, the compositions of the invention further comprise an adjuvant capable of associating with the lipopolyamine/nucleic acid complex and of improving the transfecting power thereof.

In this respect, the compositions of the invention may comprise one or more neutral lipids as adjuvants. Such compositions are particularly advantageous, especially when the ratio R is low.

More preferably, the neutral lipids used in the context of the present invention are lipids containing 2 fatty chains.

Advantageously, natural or synthetic lipids, which may be zwitterionic or devoid of ionic charge under the physiological conditions, are used. They may be chosen more particularly from dioleoylphosphatidylethanolamine (DOPE), oleoylpalmitoylphosphatidylethanolamine (POPE), di-stearoyl, -palmitoyl, -myristoyl phosphatidylethanolamine as well as derivatives thereof N-methylated 1 to 3 times, phosphatidylglycerols, diacylglycerols, glycosyldiacylglycerols, cerebrosides (such as galactocerebrosides in particular), sphingolipids (such as sphingomyelins in particular) or alternatively asialogangliosides (such as asialoGM1 and GM2 in particular).

These various lipids may be obtained either by synthesis or by extraction from organs (example: the brain) or from eggs, by standard techniques well known to those skilled in the art. In particular, the extraction of natural lipids may be performed using organic solvents (see also Lehninger, Biochemistry).

The preferred neutral lipid is DOPE.

A preferred pharmaceutical composition of the invention comprises interfering RNAs, preferably siRNAs, silencing IL-1β, IL-6 and IL-18, wherein the iRNAs are complexed with cationic liposome(s) comprising RPR209120 and DOPE.

### DNA carrier:

Any DNA molecule can be used as a carrier. Preferably the composition comprises a DNA molecule of at least 800bp, or at least 1kb, preferably, between 1kb and 3kb.

Advantageously one may use a ratio DNA/iRNA of at least 1 (for instance 10µg iRNA+10µg DNA), or preferably a ratio DNA/iRNA between 1 and 4, or most preferably between 1 and 2.

### Production of Lipoplexes :

The iRNAs, either separately or all combined together, are associated with the lipopolyamine to form a lipopolyamine/iRNA complex.

In order to obtain a maximum effect for the compositions of the invention, the respective proportions of the lipopolyamine and of the nucleic acid are preferably determined such that the ratio R of positive charges in the lipopolyamine considered to negative charges in the said nucleic acid is optimal. Since this optimal ratio varies in particular according to the mode of use, namely in vivo or in vitro, and according to the cell type to be transfected, it is optimized for each particular case. This optimization falls within the competence of a person skilled in the art. The ratio of lipopolyamine/iRNA may be of about 0,1 to 20nmoles, preferably 1 to 10, most preferably about 6nmoles of lipopolyamine for 1µg of nucleic acids.

More generally, it may be desired to use lipopolyamine with charges in a ratio of 2 to 5, with a length of 500 to 700nm.

When used, the adjuvant combines with the lipopolyamine/iRNA complex. The formation of lipoplexes is achieved, e.g. as described in Frisch, B. et al. 2004. When a neutral lipid is used as an adjuvant, one may use a ratio of neutral lipid/lipopolyamine of 1/1. In a particular embodiment, equal volume of liposome DOPE+RPR209120 (60 nmoles) and nucleic acids (10 µg iRNA ± 10µg cDNA) were mixed and incubated for 30 min. at room temperature before injection.

### Formulations and uses:

A further subject of the invention is the use of interfering RNAs (iRNAs) silencing IL-1β, IL-6, IL-18 and/or IL-17 in combination with a lipopolyamine, for the preparation of a medicament for preventing or treating an inflammatory disorder.

Another subject of the invention is a method for preventing or treating an inflammatory disorder in a patient in need thereof, which method comprises administering to said patient interfering RNAs (iRNAs) silencing IL-1β, IL-6, IL-18 and/or IL-17 in combination with a lipopolyamine.

The patient is preferably a human, but may also be a mammal including rodents, sheep, cattle, cats, dogs, horses, monkeys etc.

The therapeutic treatment includes a prophylactic or curative treatment. It extends from partial alleviation of at least of the symptoms of the inflammatory disorder to complete remission or cure of the disorder.

The duration of the combination therapy depends on the type of inflammatory disorder being treated, the age and condition of the patient, the stage and type of the patient's disease, and how the patient responds to the treatment. Additionally, a person having a greater risk of developing an inflammatory disorder (e.g., a person who is genetically predisposed or previously had an inflammatory disorder) may receive prophylactic treatment to inhibit or delay an inflammatory response.

The infammatory disorder may be selected from the group consisting of rheumatoid arthritis, Crohn's disease, psoriasis, psoriatic rhumatism, juvenile arthritis and spondylarthitis. The inflammatory disorder may also include other autoimmune diseases, such as asthma, multiple sclerosis, systemic lupus erythematosus, scleroderma, systemic sclerosis, or Sjogren's syndrome.

The compositions of the invention are particularly useful for preventing or treating joint inflammation.

In a particular embodiment, the inflammatory disorder is rheumatoid arthritis, and the iRNAs, preferably siRNAs, are complexed with cationic liposome(s) comprising RPR209120 and DOPE.

The iRNAs in combination with a lipopolyamine may be in the form of a single formulation. Alternatively they may be in the form of separate compositions, each comprising an iRNA silencing at least one cytokine among IL-1β, IL-6, IL-18 and/or IL-17, and said separate compositions are to be administered simultaneously or sequentially. For that prospect, it can be provided a kit comprising at least two among :
(i) a pharmaceutical composition comprising interfering RNAs silencing IL-1β in combination with a lipopolyamine;
(ii) a pharmaceutical composition comprising interfering RNAs silencing IL-6 in combination with a lipopolyamine;
(iii) a pharmaceutical composition comprising interfering RNAs silencing IL-18 in combination with a lipopolyamine; or
(iv) a pharmaceutical composition comprising interfering RNAs silencing IL-17 in combination with a lipopolyamine;

A preferred kit comprises:
(i) a pharmaceutical composition comprising interfering RNAs silencing IL-1β in combination with a lipopolyamine;
(ii) a pharmaceutical composition comprising interfering RNAs silencing IL-6 in combination with a lipopolyamine; and
(iii) a pharmaceutical composition comprising interfering RNAs silencing IL-18 in combination with a lipopolyamine.

The compositions according to the invention may be formulated for the purpose of topical, cutaneous, oral, rectal, vaginal, parenteral, intranasal, intravenous, intramuscular, subcutaneous, intraoccular, transdermal, etc. administration. The pharmaceutical compositions of the invention preferably contain a vehicle which is pharmaceutically acceptable for an injectable formulation, for systemic administration or for direct injection into the desired organ. They may in particular be sterile, isotonic solutions or dry compositions, in particular freeze-dried compositions, which, by addition depending on the case of sterilized water or of physiological saline, allow injectable solutions to be made up.

In a preferred embodiment, the medicament is in a suitable form for an intravenous injection.

The invention is further illustrated by the Figures and the below Examples, which do not limit the scope of the invention.

### FIGURE LEGENDS

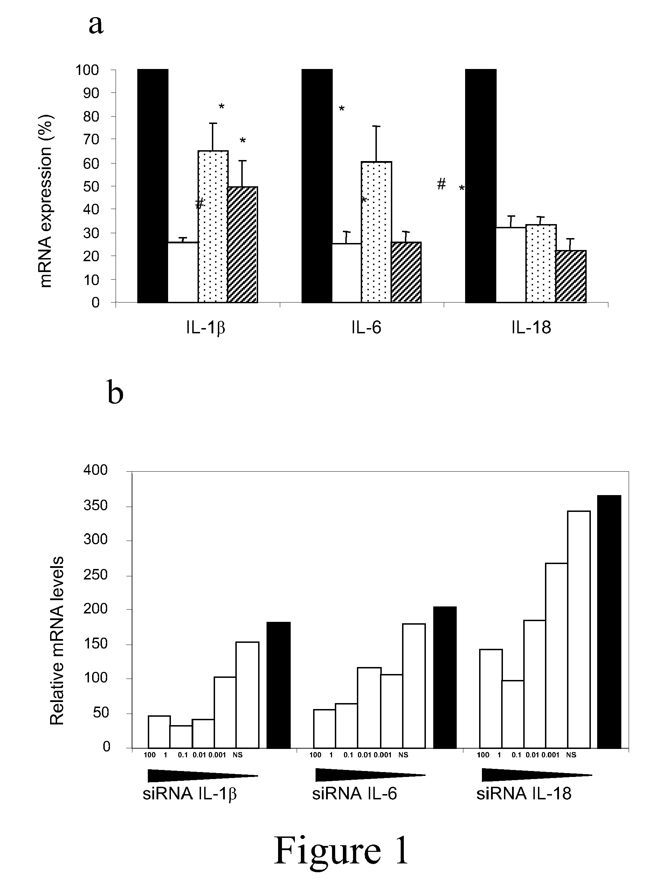

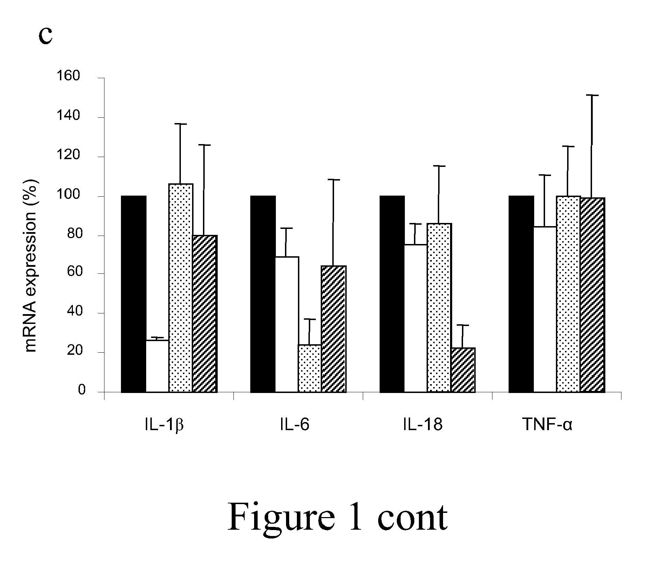

**Figure 1****: In vitro validation of siRNA sequences specifically designed to target mouse IL-1β, IL-6 and II-18.** Two different sequences named A and B were designed for each targeted cytokines using the siDesign software from Dharmacon. The inhibition of targeted mRNA was assessed by quantitative RT-PCR following LPS challenge of the mouse macrophage cell line J774.1 as described in M&M. An irrelevant siRNA was used as control. Data are representative of 2-3 independent experiments. (**a**) Total RNA were obtained from cells incubated with 150 nM of an irrelevant siRNA (black bars), sequence A (empty bars), sequence B (dotted bars) or mixed siRNA sequences A and B (hatch bars) formulated with lipofectamine. The mRNA levels of IL-1β, IL-6 and IL-18 were measured by QRT-PCR. Values were normalized to GAPDH and presented as a percentage of control-siRNA. The values represent means ± SEM of pooled triplicates. #p=0.014 and *p=0.037. (**b**) The J774.1 cells were transfected with increasing concentrations of sequence A siRNAs (0.001, 0.01, 0.1, 1 or 100 nM) and the respective mRNA levels of IL-1β, IL-6 and IL-18 were measured by QRT-PCR in pooled triplicates. The relative cytokine/GAPDH mRNA levels at each dose are represented. Black bars represent J774.1 cells transfected with 100 nM of irrelevant siRNA. (c) The mRNA levels of IL-1β, IL-6, IL-18 and TNF-α were measured by QRT-PCR following pooled duplicates of J774.1 lipofected with 150 nM of IL-1β (empty bars), IL-6 (dotted bars), or IL-18 (hatch bars) sequence A siRNAs and LPS challenge. Values were normalized to GAPDH mRNA levels and presented as a percentage of control-siRNA.

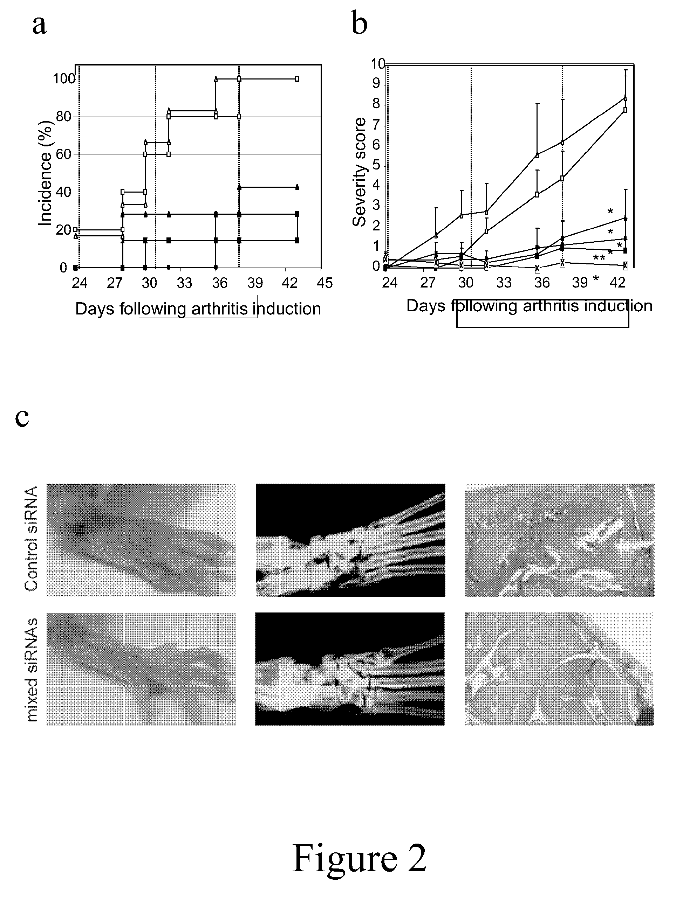

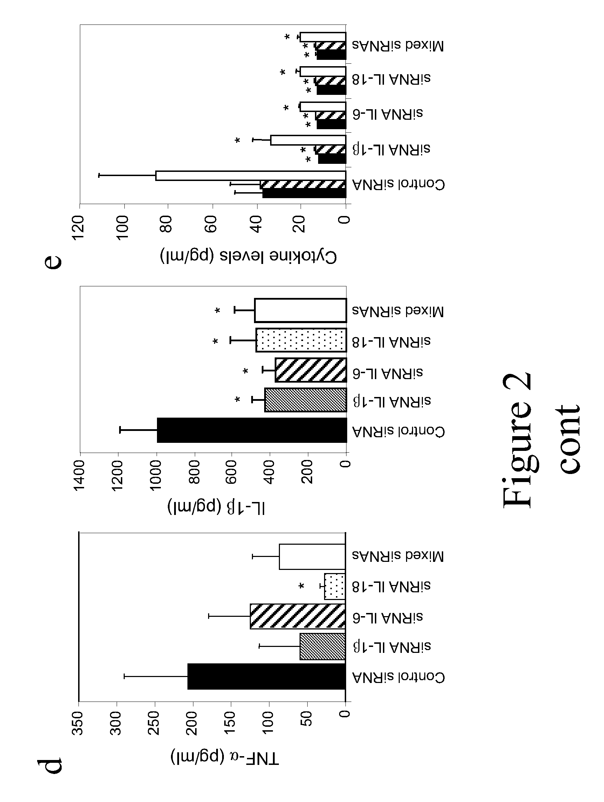

**Figure 2****: Comparative study of systemic delivery of siRNA lipoplexes targeting different pro-inflammatory cytokines in experimental arthritis.** DBA/1 mice were immunized with bovine type II collagen and boosted on day 21. From day 24, 10 µg of the sequence A siRNA IL-1β (▲), IL-6 (■), or IL-18 (●) were injected intravenously alone or mixed (x) once a week (dotted lines). The control groups were either injected with saline buffer (Δ) or an irrelevant siRNA sequence (□). All siRNAs were formulated with the cationic liposome RPR209120 and a DNA carrier as described in M&M. (**a**) Hind paw swelling and inflammation of the four limbs were determined for each mouse and the percentage of the affected animals were plotted at the indicated time for each group. (**b**) Paw swelling was measured with a calliper for each mouse during disease course, and the severity of arthritis was graded according to the scale defined in M&M. The mean value ± SEM were represented for each group (n=5-7 mice/group). (**c**) Macroscopic changes after the administration of siRNA and representative radiology and histology of mice paws following systemic siRNA treatments. (**d**) Pro-inflammatory cytokine levels in the 24 hrs knee-conditioned media from the different groups were analysed by ELISA. (**e**) MCP-1 (black bars), TNF-α (hatch bars) and IL-6 (white bars) serum levels were measured by CBA flex kit. These results are representative of two independent experiments. *p≤0,05, **p≤0,01, ***p≤0,001 compared with control siRNA-injected group.

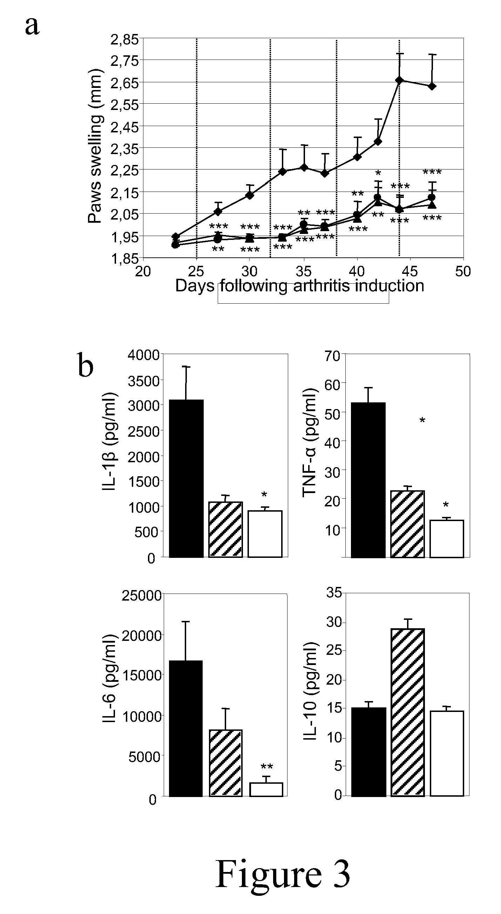

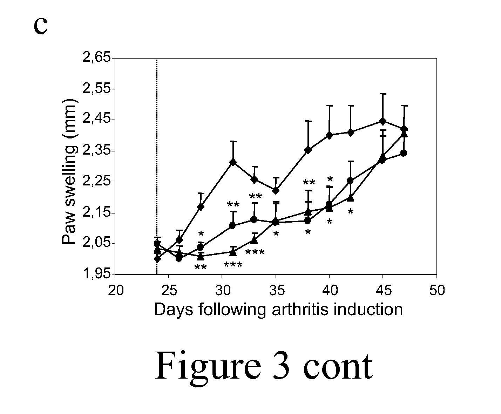

**Figure 3****: Comparison of the protective effect of the combined pro-inflammatory cytokine siRNAs on arthritis with the TNF-α siRNA therapeutic potency.** DBA/1 mice were immunized with bovine type II collagen and boosted on day 21. The TNF-α siRNA (●, hatch bars) or mixed IL-1β/IL-6/IL-18 sequence A siRNAs (▲, white bars) were complexed with the cationic liposome RPR209120 and a DNA carrier as described in M&M, and repetitively injected intravenously (10 µg, dotted lines) every 7 days from day 25 (**a**) or only once on day 24 (**c**). The control groups were injected with an irrelevant siRNA sequence (◆, black bars). (**a**) Paw swelling was measured with a calliper for each mouse during disease course and the plotted at the indicated time for each group as the mean value ± SEM (n=8 mice/group). (**b**) At euthanasia, mouse knee joints were collected for each group of treatment and incubated 24 hrs into culture medium. Pro- and anti-inflammatory cytokine levels were measured by CBA flex kit (IL-10 and TNF-α) or ELISA (IL-1β and IL-6) in the knee-conditioned media from treated mice. (**c**) Paw swelling was measured for each mouse during disease course, and the mean value ± SEM were represented for each group (n=9 mice/group). *p≤0,05, **p≤0,01, ***p≤0,001 compared with control siRNA-injected group.

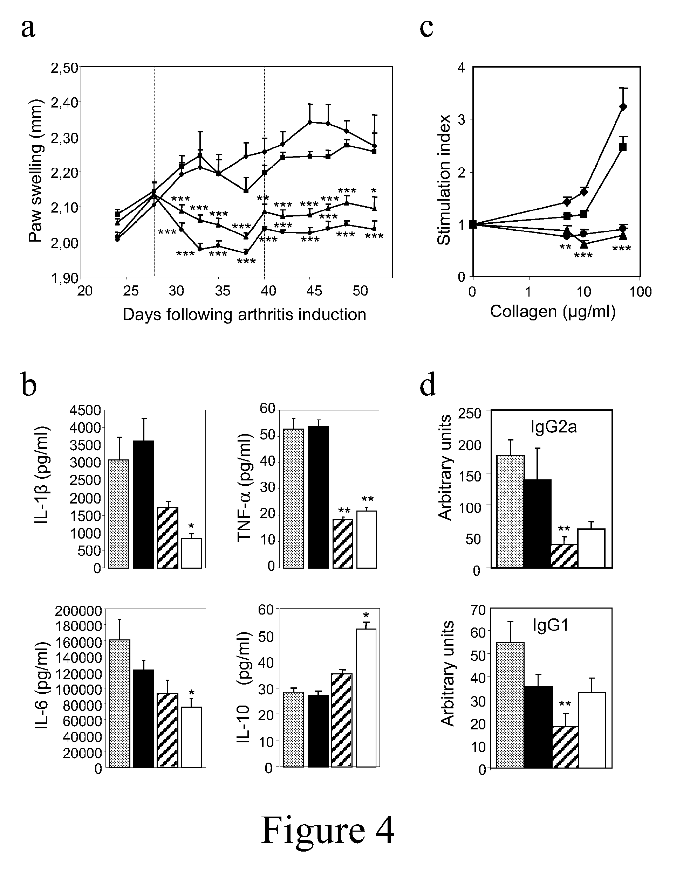

**Figure 4****: Therapeutic effect of the combined pro-inflammatory cytokine siRNAs on established arthritis.** DBA/1 mice were immunized with bovine type II collagen and boosted on day 21. The TNF-α siRNA (▲,hatch bars) or mixed IL-1β/IL-6/IL-18 sequence A siRNAs (●,white bars) formulated with the cationic liposome RPR209120 and a DNA carrier were injected intravenously (10 µg, dotted lines) on day 28, when more than 70% of the mice showed macroscopic signs of arthritis, and on day 40 when the protective effect relapses (n=9-10 mice/group). The control groups were either injected with saline buffer (◆, grey bars) or an irrelevant siRNA sequence (■, black bars). (**a**) The paw swelling was measured for each mouse during disease course with a calliper, and the mean values ± SEM were represented overtime for each group. (**b**) At euthanasia, pro- and anti-inflammatory cytokine levels were measured by specific ELISA (IL-1β and IL-6), or CBA flex kit (TNF-α and IL-10) in the 24hrs knee-conditioned media for each mice and the mean value ± SEM was represented for each group of treatment (**c**) Spleen cells from the different groups were stimulated with increasing concentrations of inactivated bCII. Cell viability was measured by the CellTiter-Glo luminescent assay 4 days later and proliferative response expressed as a stimulation index. (**d**) The bCII-specific IgG1 and IgG2a antibody levels in sera collected 41 days following immunization were quantified by ELISA. Data are presented as the mean ± SEM using arbitrary units, as described in M&M. These results are representative of two independent experiments. *p≤0,05, **p≤0,01, ***p≤0,001 compared with control siRNA-injected group.

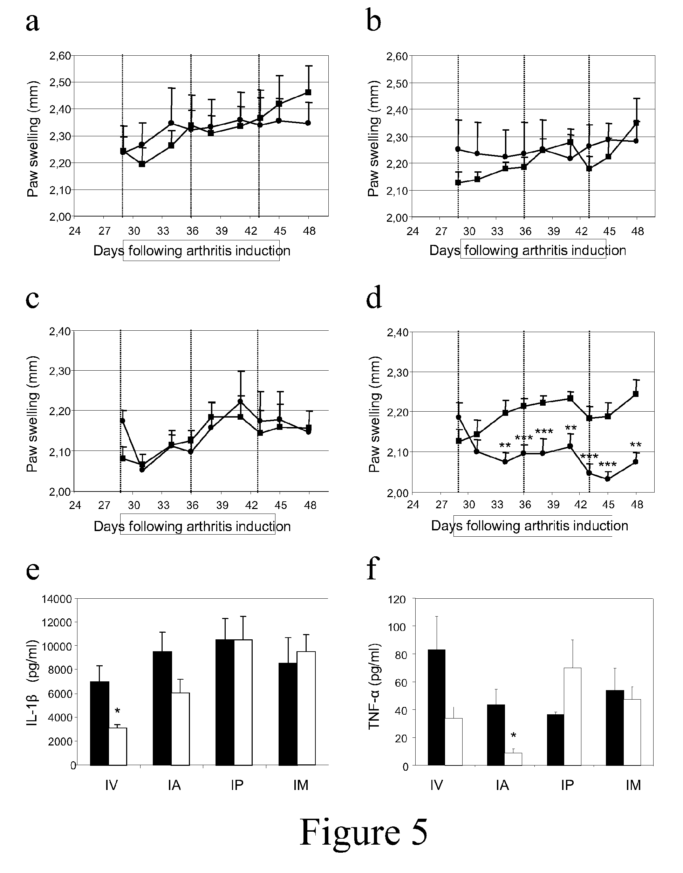

**Figure 5****: Prevention of arthritis by the combined pro-inflammatory cytokines siRNA/cationic liposome complex using different routes of delivery.** DBA/1 mice were immunized with bovine type II collagen and boosted on day 21. From day 29, 10 µg of the mixed IL-1β/IL-6/IL-18 sequence A siRNAs (●, white bars), or an irrelevant siRNA sequence (■, black bars), formulated with the cationic liposome RPR209120 and a DNA carrier were injected once a week (dotted lines) either intraperitoneally (**a**), intramuscularly into the anterior tibialis muscle (**b**), intra-articularly into both knee joints (**c**), or intravenously into the tail vein (**d**). The paw swellings were measured 3 times a week until day 48 for each mouse, and the mean values ± SEM were represented overtime for each group. At euthanasia, IL-1β (**e**) and TNF-α (**f**) levels were measured by specific ELISA or CBA respectively in the 24hrs knee-conditioned media from the different treated groups. *p≤0,05, **p≤0,01, ***p≤0,001.

### EXAMPLE 1: IL-1β, IL-6 and IL-18 siRNAs

### METHODS

*Cell lines and reagents.* The synthetic IL-1β, IL-6 and IL-18 siRNAs were purchased from CureVac (Tubingen, Germany). The two complementary RNA duplexes of each target were hybridized according to supplier's recommendations. The two sense siRNAs sequences of each cytokine were :
siRNA IL-1βA, 5'-UCAACAAGAUAGAAGUCAAdTdT-3' (SEQ ID NO: 1);
siRNA IL-1βB, 5'-UAAUUGACUUCACCAUGGAdTdT-3'(SEQ ID NO: 2);
siRNA IL-6A, 5'-CUACCAAACUGGAUAUAAUdTdT-3' (SEQ ID NO: 3);
siRNA IL-6B, 5'-AUGCUCUCCUAACAGAUAAdTdT-3' (SEQ ID NO: 4);
siRNA IL-18A, 5'-CAACACGCUUUACUUUAUAdTdT-3' (SEQ ID NO: 6);
and siRNA IL-18B, 5'-UUCGAGGAUAUGACUGAUAdTdT-3' (SEQ ID NO: 7).

DOPE (1,2-dioleoyl-*sn*-glycerol-3-phosphoethanolamine) was purchased from Avanti Polar Lipids (Alabaster, USA). Cationic lipid RPR209120 (2-{3-[Bis-(3-amino-propyl)-amino]-propylamino}-N-ditetradecylcarbamoylmethyl-acetamide) was prepared as described (Byk et al, 2001). The lipoplexes were formed at room temperature for at least 30 min as previously published (Frisch et al, 2004). siRNA and/or DNA carrier, a pcDNA3 plasmid (Invitrogen, France), were suspended in 150 mM NaCl and mixed with an equivalent volume of saline liposome suspensions prepared at the appropriate concentrations as previously described (Khoury et al, 2006). The murine macrophage J774.1 cells were transfected in 24-well plates using siRNA duplexes with Lipofectamine as recommended (Invitrogen). The siControl non targeting siRNA was from Dharmacon (Perbio France). The medium was replaced by RPMI 1640 containing 10% heat-inactivated fetal calf serum, 100 units/ml penicillin, 100 µg/ml streptomycin, and 1 µg/ml LPS (Sigma). Transcriptional stop was made by adding 5 µg/ml of actinomycin D (Sigma). Supernatants and cells were harvested after 2h and stored for ELISA and Q-PCR respectively.

*Real-time quantitative RT-PCR.* Total RNA was extracted from LPS-treated J774A.1 cells using the RNeasy mini kit (Qiagen) following the manufacturer's protocol and quantified by spectrophotometry (Eppendorf, France). One microgram of total RNA was reverse transcribed using the Multiscribe reverse transcriptase (Applied Biosystems, Courtaboeuf, France). The assays-on-demand primers specific for mouse IL-1β, IL-6, IL-18, TNF-α and GAPDH, and the Taq Man Universal Master Mix were used according to the manufacturer's recommendations (Applied Biosystems). Measurement and analysis of gene expression were performed using the ABI Prism 7000 Sequence Detection System software. Content of cDNA samples was normalized by subtracting the number of copies of the endogenous GAPDH reference gene to the target gene (ΔCt = Ct of target gene - Ct of GAPDH). Expression of the specific gene was calculated as the difference (ΔΔCt) between normalized Ct values (ΔCt) of siRNA-treated cells versus siRNA-untreated cells. Values are expressed as the fold increase in specific gene expression as obtained using the formulae 2^{-(ΔΔCt)}.

### Arthritis induction.

Specific pathogen-free DBA/1 mice (Harlan France) were bred in our facilities and used at the age of 8-10 weeks. Collagen-induced arthritis (CIA) was induced as previously described (Perez et al, 2002) using bovine type II collagen (bCII) from MD Biosciences (Zurich, Switzerland), acetic acid from Sigma-Aldrich (l'Isle d'Abeau, France), and Freund's adjuvants from Pierce (Bezons, France). Following arthritis induction, paw thickness was measured 3 times a week with a micrometer Mitutoyo (Sigma) and arthritis was scored by macroscopic examination as previously published (Djouad et al, 2005). Equal volume of liposome RPR209120/DOPE (60 nmoles) and nucleic acids (10 µg siRNA ± 10µg cDNA) were mixed and incubated for 30 min. The irrelevant siRNA with random nucleotides and no known specificity (Laderach et al, 2003). When clinical signs of arthritis appeared, mice were randomised and injected intravenously either with the saline buffer alone, formulation alone or complexed with siRNA. At euthanasia, the hind paws were collected for radiography with a Kodak Faxitron system (Kodak France), scored and processed for overall histo-pathological analysis as previously described (Appareilly et al, 1998). Severe arthritis were considered for radiological or histological scores over 2. Approval for these studies was obtained from the Ethic committee on Animal Research of the Languedoc-Roussillon (CE-LR-0505).

*Cytokines and anti-type II collagen assays.* Serum samples were obtained by retro-orbital puncture on anaesthetized animals at indicated times. At day of sacrifice, patellaes were collected and incubated 24h in RPMI (300 µl). Supernatants were stored at -20°C until assayed. The mIL-1β, mIL-6 and mTNF-α secretions were measured by specific ELISAs (CliniSciences, Montrouge, France), and the secretion of murine IL-10, IL-12, IFN-γ and MCP-1 were determined using Cytometric Bead Array (BD Biosciences, France). Spleens and draining lymph nodes were collected at sacrifice and cultured at 2 x 10⁶ cells/well as previously published (Djouad et al, 2005). Supernatants were harvested for murine IFN-γ and IL-4 quantification using specific ELISA (CliniSciences). Serum level of antibodies against type II collagen was measured by ELISA as previously reported (Perez et al, 2002). Antibody units were determined using a reference serum created from pooled sera of arthritic mice and assigned an arbitrary level of bCII-specific antibodies.

*Statistics.* Statistical analysis was done using Fisher's exact test for contingency group comparisons, and unpaired t test or Mann-Whitney test as appropriate according to data distribution. All data were analyzed by the program Instat2.1 for Macintosh.

### RESULTS

### Designing siRNAs against pro-inflammatory cytokine mRNAs

Two siRNA sequences (named A and B) were designed for three different cytokines having key pro-inflammatory properties in RA, i.e. IL-1β, Il-6 and IL-18.

Since these cytokines are all macrophage-derived, the mouse macrophage cell line J774.1 was used to examine the ability of the different siRNAs to reduce their respective mRNA targets upon LPS challenge. RNAi being a post-transcriptional gene-silencing process, the inhibition was assessed at the mRNA level by QRT-PCR and an irrelevant siRNA sequence was used as control to verify down-regulation specificity (**Fig. 1**). Cells transfected with the siRNA sequences A showed a 70-75% decrease of the LPS-induced IL-1β, IL-6 and IL-18 mRNA levels compared with the control siRNA (**Fig. 1a**). Inhibition of the three targeted mRNA was similar even less efficient using the siRNA sequences B, or mixing both A and B sequences. The silencing obtained with the sequence A was dose-dependent and maintained for all 3 targets when lowering their respective siRNA concentrations to 1nM (**Fig. 1b**). No difference was observed between the transfected cells with or without the control siRNA (data not shown). The down-expression of each targeted cytokine is specific as anti-IL-1β siRNA did not modify IL-6, IL-18 or TNF-α mRNA expression, and anti-IL-6 siRNA or anti-Il-18 siRNA did not modify other pro-inflammatory cytokines induced by LPS challenge (**Fig. 1c**).

### In vivo gene silencing of pro-inflammatory cytokines prevents arthritis

In RA, macrophage-derived pro-inflammatory cytokines participate to joint inflammation and tissue destruction. The inventors thus investigated the therapeutic potential of the inhibitory effect of the anti-IL-1β; anti-IL-6 and anti-IL-18 siRNA sequences validated in vitro (sequence A) in a mouse model of RA. After the collagen boost, DBA/1 mice were injected intravenously with 0,5mg/kg of siRNA separately or mixed, weekly over one month. The anti-cytokine siRNAs were formulated with the cationic liposome RPR209120 and a DNA carrier as previously described (Khoury et al, 2006). Formulated irrelevant siRNA and saline buffer were used as controls (**Fig. 2**). Systemic treatment with the formulated targeting siRNAs showed delayed onset, lower incidence and decreased severity of CIA in comparison with control groups, as assessed by clinical scores and paw swellings (**Fig. 2a,b**). When treated with the anti-IL-1β, anti-IL-6 or anti-IL-18 siRNAs, only 28 or 43 % of mice develop arthritis, compared with the control groups that develop arthritis with 100% of incidence. Importantly, combining all three siRNA-lipoplexes together, a higher protection was observed than all groups (only 1 mouse out of 7 developed mild arthritis). Radiological examination of paws recovered from each group at euthanasia correlated with clinical observations with median radiological scores of 1.25 for the IL-1β-, 0.75 for the IL-6-, 1 for the IL-18- and 0.5 for the mix siRNA-treated groups, compared with 1.5 in the controls. The histopathological analyses were in line with these results, with the most important reduction of inflammation of synovial tissue (infiltration of mononuclear cells into joint cavity), pannus formation (synovial hyperplasia), cartilage and bone destruction in the mix siRNA-treated groups (**Fig. 2c**). The therapeutic effect observed in all siRNA treated groups was associated with a decrease of the TNF-α (40-87%) and IL-1β (75-82%) levels in the knee-conditioned media (**Fig. 2d**), and with a strong reduction of the circulating IL-6 (60-75%), TNF-α and MCP-1 (dropping from 38 ± 13 and 37 ± 12 pg/ml respectively to barely detectable levels) (**Fig. 2e**). No variation of the systemic levels of IL-4, IL-10 or IFN-γ was detected according to treatment (data not shown). Considering overall clinical and biological parameters, a preferential protection was observed when using the three combined anti-IL-1β/IL-6/IL-18 siRNAs (tri-therapy).

### siRNA tri-therapy is an alternative to anti-TNF to prevent CIA

For a better evaluation of this new therapeutic approach a comparison was performed with the previously validated siRNA TNF-α treatment. Following the same experimental procedure, both treated groups showed a delayed onset (40, 38 and 29 days for siRNA mix, siRNA TNF-α and siRNA control, respectively), a lower incidence (25, 63, and 100% of arthritic mice) and a decreased severity of CIA (**Fig. 3a**) in comparison with the irrelevant siRNA-injected group. Local pro-inflammatory cytokine levels decreased in both treated groups, with a higher inhibition of IL-1β, IL-6 and TNF-α secretion in the tri-therapy siRNA-injected group (**Fig. 3b**). Although we observed an increase of local IL-10 secretion only in the TNF-α siRNA injected group, all the clinical and biological data taken together suggest a better benefit for the combined siRNA treatment. To evaluate the duration of a single systemic administration of the siRNA lipoplexes, CIA mice were injected once on day 24 with either the TNF-α siRNA or with the mixed anti-IL-1β/IL-6/IL-18 siRNA lipoplexes (**Fig. 3c**). The maximum effect observed was on day 4 for both treatments, but the suppressive effect of the tri-therapy on arthritis continued until day 9 post-injection while it was reversed on day 7 in the TNF-α siRNA-treated group. However, in both anti-inflammatory siRNA-lipoplex-based therapies clinical signs of arthritis remained less severe than in controls for 16 days. Based on these findings, continuous cytokine suppression might be obtained with administration of the siRNA lipoplexes every week.

### Systemic delivery of siRNA-lipoplexes tri-therapy is potent to treat established arthritis

As a way to mimic the late stage of arthritis in patients, the inventors next tested if such a systemic cytokine silencing approach could be beneficial for established arthritis. When more than 70% of the mice showed macroscopic signs of arthritis we administered either the anti-IL-1β/IL-6/IL-18 siRNA or the anti-TNF-α siRNA lipoplexes intravenously (**Fig. 4**). Mice treated with the siRNA lipoplexes targeting anti-inflammatory cytokines showed a decreased severity of CIA in comparison with saline-treated or irrelevant siRNA-treated arthritic mice, as assessed by clinical scores and paw swellings (**Fig. 4a**). Although a single administration of the TNF-α siRNA was enough to significantly ameliorate the pathological signs of arthritis, the tri-siRNA therapy offered a significantly best protection against disease (p<0.05 from day 33 to day 38). The therapeutic effect was maximal till 10 days post-injection. On day 40 following immunization, when mice relapse, the inventors re-administered the corresponding siRNAs regimen in the different groups. The inventors saw no remission in therapeutic effects two weeks after cessation of siRNA injection (day 40), indicating that no additional siRNA is necessary after a short period of treatment to maintain protection from the disease. The present data suggest that both TNF-α and mixed IL-1β/IL-6/IL-18 siRNA lipoplexes blocked disease development and led to a stabilization in clinical scoring and incidence of arthritis.

### Anti-inflammatory siRNAs regulate immunological balance in CIA

The mechanisms underlying the decrease in CIA severity following anti-inflammatory siRNA treatments were next investigated. First, to test whether the local pro/anti-inflammatory cytokine balance was reversed by the siRNA injections, secretion levels of IL-1β, IL-6, TNF-α and IL-10 were analysed in knee-conditioned media collected at euthanasia. Knee joints from mice injected with the siRNA control showed low levels of the anti-inflammatory IL-10 cytokine, but high production of pro-inflammatory IL-1β, IL-6, and TNF-α cytokines (**Fig. 4b**). In contrast, mice injected with the therapeutic siRNA-lipoplexes had decreased local secretion of IL-1β, IL-6, and TNF-α and increased production of IL-10, more pronounced in the tri-therapy group. Second, to test whether impaired T-cell functions in siRNA-treated mice lead to CIA inhibition, the collagen-specific proliferative responses of spleen cells from CIA mice was analysed. Whereas spleen cells from irrelevant siRNA-treated mice proliferated similarly to control mice in response to bCII, T cells from mice receiving both anti-inflammatory siRNA treatments did not respond to bCII (**Fig. 4c**). The proliferative response was similar in all groups following non-specific stimulation with ConA (data not shown). These data indicate that TNF-α or mixed IL-1β/IL-6/IL-18 siRNA-lipoplexes administration during CIA development inhibits T-cell clonal expansion in response to antigenic challenge.

Finally, high levels of circulating antibodies directed against bCII accompany the development of CIA and determine disease susceptibility. The serum levels of bCII-specific IgG2a and IgGlwere measured during disease course as a reflection of the Th1 and Th2 switch. CIA resulted in high levels of bCII-specific IgG antibodies, characterized by a high IgG2a/IgGl ratio that was not modified following administration of irrelevant siRNA (ratio median of arbitrary units were respectively 3.5 and 4.0). In contrast, treatment of arthritic mice with anti-inflammatory siRNA-lipoplexes reduced the IgG2a/IgG1 ratio (2.6 in TNF-α siRNA-treated group and 1.5 in the mixed IL-1β/IL-6/IL-18 siRNA-injected group, p<0.05), mainly attributable to the bCII-specific IgG2a inhibition (**Fig. 4d**). All these data indicate that both anti-inflammatory siRNA treatments have in vivo immuno-modulatory effects including inhibition of antigen-specific T-cell proliferation and regulation of Th1/Th2 balance.

### The best delivery route of siRNA-lipoplexes is by intravenous administration

To determine whether another route of delivery for the siRNA-lipoplexes would be efficient in CIA, irrelevant siRNA or the mixed IL-1β/IL-6/IL-18 siRNA were formulated and injected once a week from disease onset (day 28), either intraperitoneally (**Fig. 5a**), intramuscularly into the anterior tibialis muscle of mice (**Fig. 5b**), or intra-articularly into both knee joints (**Fig. 5c**), and compared with intravenously administration into the tail vein (**Fig. 5d**). Except from intravenous administration, none of the other routes tested for siRNA-lipoplex injection showed a therapeutic potential as assessed by clinical score (data not shown) and paw swellings (**Fig. 5a,b,c,d**). These clinical observations were associated with no variation of the local pro-inflammatory cytokine production in intraperitoneally and intramuscularly injected groups, as assessed by measure of IL-1β and TNF-α secretion in knee-conditioned media at euthanasia (**Fig. 5e****,f**). Interestingly, although IL-1β local production was only decreased following intravenous administration of the therapeutic siRNA-lipoplexes, the TNF-α levels were decreased to similar extends following intra-articular and intravenous siRNA-lipoplexes delivery (60-80% inhibition).

### CONCLUSION:

The IL-1β, IL-6 or IL-18 siRNA injection showed a high protective effect against experimental model of arthritis, that was enhanced when combined all 3 together. Main targeted organs by siRNA administration were liver and spleen, the addition of the RPR290120/DOPE liposome and carrier DNA significantly increased the organs uptake.

### EXEMPLE 2:Il-17 siRNA

### METHODS:

The inventors screened siRNA sequences targeting the murine IL-17A mRNA. For that purpose 8.10⁵ cells / well of murine fibroblasts (NIH cells) were deposited in 6-well plate. Cells were co-transfected with an expression plasmid encoding the IL-17A gene or a control plasmid, and a siRNA against either mIL-17A or no specific target (siRNA CT).

Transfection conditions were as follows:
7,5 µl Lipofectamine / 250 µl of Opti-MEM / well
Plasmids used:
   pACMV-IL17A, 2µg / well
   pCDNA3 (empty plasmid as negative control)
siRNA concentrations : 10nM and 100nM
Vf = 2ml

After an over-night ransfection, cells and supernatants were collected. mRNA was extracted using QIAGEN extraction kit, mRNAs were reverse transcribed and cDNA were analyzed by QPCR IL-17 secretion was evaluated by ELISA (R&D protocol)

Nb: Experiment was done three times in triplicates conditions.

### RESULTS:

A 60% inhibition of the IL-17 mRNA expression was observed when using the sequence SEQ ID NO:8 at 100nM.

These results were confirmed at the protein level following IL-17 detection in supernatants by ELISA.

### REFERENCES

Apparailly, F. et al. Adenovirus-mediated transfer of viral IL-10 gene inhibits murine collagen-induced arthritis. J Immunol 160, 5213-20 (1998).
Byk, G., Scherman, D., Schwartz, B. & Dubertret, C. Lipopolyamines as transfection agents and pharmaceutical uses thereof. in U.S. Patent 6 171 612 (2001).
Bloquel, C., Bessis, N., Boissier, M.C., Scherman, D. & Bigey, P. Gene therapy of collagen-induced arthritis by electrotransfer of human tumor necrosis factor-alpha soluble receptor I variants. Hum Gene Ther 15, 189-201 (2004).
Caplen, N.J. Gene therapy progress and prospects. Downregulating gene expression: the impact of RNA interference. Gene Ther 11, 1241-8 (2004).
Choy, E.H. et al. Therapeutic benefit of blocking interleukin-6 activity with an anti-interleukin-6 receptor monoclonal antibody in rheumatoid arthritis: a randomized, double-blind, placebo-controlled, dose-escalation trial. Arthritis Rheum 46, 3143-50 (2002).
Dayer, J.M., Graham, R., Russell, G. & Krane, S.M. Collagenase production by rheumatoid synovial cells: stimulation by a human lymphocyte factor. Science 195, 181-3 (1977).
Dinarello, C.A. et al. Tumor necrosis factor (cachectin) is an endogenous pyrogen and induces production of interleukin 1. J Exp Med 163, 1433-50 (1986).
Dinarello, C.A. & Moldawer, L.L. Proinflammatory and anti-inflammatory cytokines in rheumatoid arthritis, 352 (Thousand oaks, 2002).
Djouad, F. et al. Reversal of the immunosuppressive properties of mesenchymal stem cells by tumor necrosis factor alpha in collagen-induced arthritis. Arthritis Rheum 52, 1595-603 (2005).
Feldmann, M., Brennan, F.M. & Maini, R.N. Role of cytokines in rheumatoid arthritis. Annu Rev Immunol 14, 397-440 (1996).
Frisch, B. et al. A new triantennary galactose-targeted PEGylated gene carrier, characterization of its complex with DNA, and transfection of hepatoma cells. Bioconjug Chem 15, 754-64 (2004).
Ghayur, T. et al. Caspase-1 processes IFN-gamma-inducing factor and regulates LPS-induced IFN-gamma production. Nature 386, 619-23 (1997).
Gould, D.J., Bright, C. & Chernajovsky, Y. Inhibition of established collagen-induced arthritis with a tumour necrosis factor-alpha inhibitor expressed from a self-contained doxycycline regulated plasmid. Arthritis Res Ther 6, R103-13 (2004).
Gracie, J.A. et al. A proinflammatory role for IL-18 in rheumatoid arthritis. J Clin Invest 104, 1393-401 (1999).
Illei, G.G. & Lipsky, P.E. Novel, non-antigen-specific therapeutic approaches to autoimmune/inflammatory diseases [In Process Citation]. Curr Opin Immunol 12, 712-8 (2000).
Inoue, A. et al. Electro-transfer of small interfering RNA ameliorated arthritis in rats. Biochem Biophys Res Commun 336, 903-8 (2005).
Taylor, P.C., Williams, R.O. & Maini, R.N. Immunotherapy for rheumatoid arthritis. Curr Opin Immunol 13, 611-6 (2001).
Karagiannis, T.C. & El-Osta, A. RNA interference and potential therapeutic applications of short interfering RNAs. Cancer Gene Ther 12, 787-95 (2005).
Khoury, M. et al. Efficient new cationic liposome formulation for systemic delivery of small interfering RNA silencing tumor necrosis factor alpha in experimental arthritis. Arthritis Rheum 54, 1867-77 (2006).
Kim, S.H. et al. Ex vivo gene delivery of IL-1Ra and soluble TNF receptor confers a distal synergistic therapeutic effect in antigen-induced arthritis. Mol Ther 6, 591-600 (2002).
Kohno, K. et al. IFN-gamma-inducing factor (IGIF) is a costimulatory factor on the activation of Th1 but not Th2 cells and exerts its effect independently ofIL-12. J Immunol 158, 1541-50 (1997).
Laderach, D., Compagno, D., Danos, O., Vainchenker, W. & Galy, A. RNA interference shows critical requirement for NF-kappa B p50 in the production of IL-12 by human dendritic cells. J Immunol 171, 1750-7 (2003).
McInnes, I.B. & Gracie, J.A. Targeting cytokines beyond tumor necrosis factor-alpha and interleukin-1 in rheumatoid arthritis. Curr Rheumatol Rep 6, 336-42 (2004).
Mello, C.C. & Conte, D., Jr. Revealing the world of RNA interference. Nature 431, 338-42 (2004).
Perez, N. et al. Tetracycline transcriptional silencer tightly controls transgene expression after in vivo intramuscular electrotransfer: application to interleukin 10 therapy in experimental arthritis. Hum Gene Ther 13, 2161-72 (2002).
Plater-Zyberk, C. et al. Therapeutic effect of neutralizing endogenous IL-18 activity in the collagen-induced model of arthritis. J Clin Invest 108, 1825-32 (2001).
Rohn et al, Eur. J Immunol. 2006, vol 36, pp 2857-67).
Schiffelers, R.M., Xu, J., Storm, G., Woodle, M.C. & Scaria, P.V. Effects of treatment with small interfering RNA on joint inflammation in mice with collagen-induced arthritis. *Arthritis Rheum* **52**, 1314-8 (2005).
Taylor, P.C. Antibody therapy for rheumatoid arthritis. *Curr Opin Pharmacol **3**,* 323-8 (2003).
Tsutsui, H. et al. IFN-gamma-inducing factor up-regulates Fas ligand-mediated cytotoxic activity of murine natural killer cell clones. *J Immunol* **157**, 3967-73 (1996).

## Claims

1. A pharmaceutical composition comprising an interfering RNA (iRNA) silencing at least one cytokine selected from the group consisting of IL-1β, IL-6, IL-18 and IL-17, in combination with a lipopolyamine.

2. The pharmaceutical composition of claim 1, wherein the lipopolyamine is represented by the general formula I 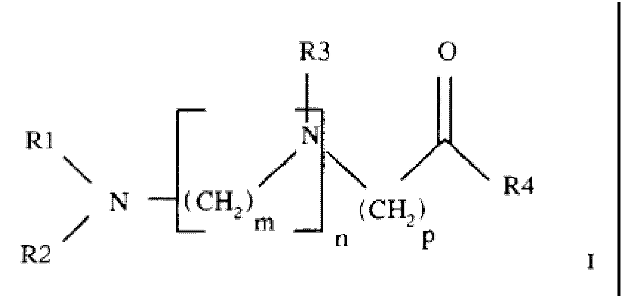 in which:
R1, R2 and R3 represent, independently of each other, a hydrogen atom or a group -(CH2)q-NRR' with
q able to range between 1, 2, 3, 4, 5 and 6, and doing so independently between the various groups R1, R2 and R3 and
R and R' representing, independently of each other, a hydrogen atom or a group --(CH2)q'-NH2, q being able to range between 1,2,3,4, 5 and 6, and doing so is independently between the various groups R and R',
m, n and p represent, independently of each other, an integer which may vary between 0 and 6 with, when n is greater than 1, m able to take different values and R3 able to take different meanings within the general formula I, and
R4 represents a group of general formula II 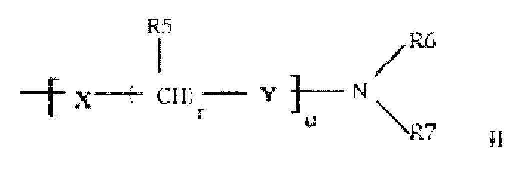 in which:
R6 and R7 represent, independently of each other, a hydrogen atom or a saturated or unsaturated C10 to C22 aliphatic radical with at least one of the two groups being other than hydrogen,
u is an integer chosen between 0 and 10 with, when u is an integer greater than 1, R5, X, Y and r able to have different meanings within the different units [X-(CHR5)r-Y]
X represents an oxygen or sulphur atom or an amine group which may or may not be monoalkylated,
Y represents a carbonyl group or a methylene group R5 represents a hydrogen atom or a side chain of a natural amino acid, which is substituted if necessary, and
r represents an integer ranging between 1 and 10 with, when r is equal to 1, R5 representing a side chain of a natural amino acid and, when r is greater than 1, R5 representing a hydrogen atom.

3. The pharmaceutical composition of claim 2 wherein the lipopolyamine is RPR209120 which is

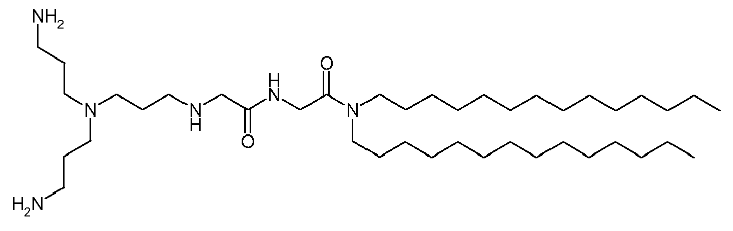

4. The pharmaceutical composition of any of claims 1 to 3, further comprising a neutral lipid.

5. The pharmaceutical composition of claim 4, wherein the neutral lipid is selected from the group consisting of dioleoylphosphatidylethanolamine (DOPE); oleoylpalmitoylphosphatidylethanolamine (POPE); distearoyl, -palmitoyl, -myristoyl phosphatidyl-ethanolamine; phosphatidylglycerols; diacylglycerols; glycosyldiacylglycerols; cerebrosides; sphingolipids; and asialogangliosides.

6. The pharmaceutical composition of any of claims 1 to 5, wherein the iRNAs are complexed with cationic liposome(s) comprising RPR209120 and DOPE.

7. The pharmaceutical composition of any of claims 1 to 6, further comprising DNA.

8. The pharmaceutical composition of any of claims 1 to 7, comprising iRNAs silencing at least two cytokines selected from the group consisting of IL-1β, IL-6, IL-18 and IL-17.

9. The pharmaceutical composition of any of claims 1 to 8, comprising iRNAs silencing IL-1β, IL-6 and IL-18 in combination with a lipopolyamine.

10. The pharmaceutical composition of any of claims 1 to 9, wherein the iRNA for silencing IL-1β is a double strand siRNA having the sequence as shown in SEQ ID NO :1.

11. The pharmaceutical composition of any of claims 1 to 10, wherein the iRNA for silencing IL-18 is a double strand siRNA having the sequence as shown in SEQ ID NO:5.

12. The pharmaceutical composition of any of claims 1 to 11, further comprising an iRNA for silencing TNF-α.

13. The pharmaceutical composition of claim 1, wherein the iRNA is in the form of a siRNA.

14. The pharmaceutical composition of claim 1, wherein the iRNA is in the form of a shRNA.

15. A kit comprising at least two among
(i) a pharmaceutical composition comprising interfering RNAs silencing IL-1β in combination with a lipopolyamine;
(ii) a pharmaceutical composition comprising interfering RNAs silencing IL-6 in combination with a lipopolyamine;
(iii) a pharmaceutical composition comprising interfering RNAs silencing IL-18 in combination with a lipopolyamine; or
(iv) a pharmaceutical composition comprising interfering RNAs silencing IL-17 in combination with a lipopolyamine.

16. Use of an iRNA silencing at least one cytokine selected from the group consisting of IL-1β, IL-6, IL-18 and IL-17, in combination with a lipopolyamine, for the preparation of a medicament for preventing or treating an inflammatory disorder.

17. The use of claim 16, wherein the medicament comprises iRNAs silencing at least two cytokines selected from the group consisting of IL-1β, IL-6, IL-18 and IL-17.

18. The use of claim 17, wherein the medicament comprises iRNAs silencing IL-1β, IL-6 and IL-18 in combination with a lipopolyamine.

19. The use of any of claims 16 to 18, wherein the inflammatory disorder is selected from the group consisting of rheumatoid arthritis, Crohn's disease, psoriasis, psoriatic rhumatism, juvenile arthritis and spondylarthitis.

20. The use of claim 19, wherein the inflammatory disorder is rheumatoid arthritis, and the iRNAs are complexed with cationic liposome(s) comprising RPR209120 and DOPE.

21. The use of any of claims 16 to 20, wherein the medicament is in a suitable form for an intravenous injection.

22. The use of any of claims 16 to 21, wherein the iRNAs in combination with a lipopolyamine are in the form of a single formulation.

23. The use of any of claims 16 to 21, wherein the iRNAs combined with a lipopolyamine are in the form of separate compositions, each comprising a iRNA silencing at least one cytokine among IL-1β, IL-6, IL-18, and IL-17 and said separate compositions are to be administered simultaneously or sequentially.

24. The use of any of claims 16 to 23, wherein the iRNA is in the form of a siRNA or a shRNA.
